# EUROPÄISCHE PATENTSCHRIFT

(11) **EP 2 379 161 B1**
(45) Veröffentlichungstag und Bekanntmachung des Hinweises auf die Patenterteilung: **07.11.2012**
(21) Anmeldenummer: 10703798.8
(22) Anmeldetag: 15.01.2010
(51) Int. Cl.: A61M 39/02, A61M 39/22, F16K 3/24, F16K 11/078, F16K 31/528

(54) **VORRICHTUNG ZUM ZUFÜHREN VON MEDIZINISCHEN FLÜSSIGKEITEN**
DEVICE FOR FEEDING MEDICAL FLUIDS
DISPOSITIF D'ADMINISTRATION DE LIQUIDES MÉDICAUX

(30) Priorität: 22.01.2009 DE 102009005822
(43) Veröffentlichungstag der Anmeldung: 26.10.2011
(73) Patentinhaber: V. Krütten Medizinische Einmalgeräte GmbH, 65510 Idstein (DE)
(72) Erfinder: HORNIG, Wolfgang, 58566 Kiespe-Bollwerk (DE); PECH, Bernhard, 65510 Hünstetten (DE)
(74) Vertreter: Richly, Erik
(86) Internationale Anmeldenummer: PCT/EP2010/000185
(87) Internationale Veröffentlichungsnummer: WO 2010/083962

(56) Entgegenhaltungen:
- EP-A1- 1 555 041
- EP-A1- 1 627 658
- FR-A1- 2 772 280
- US-A- 5 370 624

## Beschreibung

Die Erfindung betrifft eine Vorrichtung zum Zuführen von medizinischen Flüssigkeiten, insbesondere zum Applizieren eines Medikaments in die Schlauchleitung eines Überleitgeräts für die enterale oder parenterale Ernährung.

Zur enteralen oder parenteralen Ernährung finden Überleitgeräte Verwendung, mit denen eine Nährlösung aus einem Behälter einem Patienten zugeführt wird. Die bekannten Überleitgeräte weisen eine Schlauchleitung mit Konnektoren auf, die einerseits mit dem Nährlösungsbehälter und andererseits mit einer Ernährungssonde verbunden wird.

Bei der enteralen oder parenteralen Ernährung ist es in einzelnen Fällen erforderlich, in die Schlauchleitung des Überleitgeräts eine medizinische Flüssigkeit zu applizieren. Zum Zuführen von Medikamenten in die Schlauchleitung eines Überleitgeräts sind verschiedene Vorrichtungen bekannt.

Die bekannten Vorrichtungen bestehen aus einem Basiskörper und einem Verschlusskörper. Der Basiskörper weist einen Einlass, an dem eine von dem Nährlösungsbehälter abgehende Schlauchleitung angeschlossen ist, und einen Auslass auf, an dem die zu dem Patienten führende Schlauchleitung angeschlossen ist. Einlass und Auslass des Basiskörpers sind über einen Durchgang miteinander verbunden, so dass die Nährlösung von dem Nährlösungsbehälter zu dem Patienten fließen kann. Zum Applizieren eines Medikaments weist der Verschlusskörper einen Einlass auf. Der Verschlusskörper ist an dem Basiskörper zwischen einer ersten Position, in der eine Flüssigkeitsverbindung zwischen dem Einlass des Verschlusskörpers und dem Durchgang des Basiskörpers unterbrochen ist, und einer zweiten Position drehbar, in der die Flüssigkeitsverbindung zwischen dem Einlass des Verschlusskörpers und dem Durchgang des Basiskörpers hergestellt ist. Während der enteralen oder parenteralen Ernährung befindet sich der Verschlusskörper in der ersten Position. Nur zum Applizieren eines Medikaments wird der Verschlusskörper in die zweite Position verdreht.
Die Vorrichtungen zum Zuführen von medizinischen Flüssigkeiten sollten verschiedenen Anforderungen genügen. Zum Einen muss gewährleistet sein, dass die Vorrichtung sicher abdichtet, so dass keine Leckagen auftreten können. Zum Anderen sollte sich der Verschlusskörper mit verhältnismäßig geringen Kräften leicht verdrehen lassen, so dass sich die Vorrichtung einfach handhaben lässt. Neben der sicheren Abdichtung und der einfachen Handhabung wird noch ein relativ geringer konstruktiver Aufwand angestrebt, um die Produktionskosten möglichst gering zu halten.

Eine bekannte Vorrichtung zum Zuführen von medizinischen Flüssigkeiten der Anmelderin zeichnet sich dadurch aus, dass der Basiskörper über ein hohlzylindrisches Ansatzstück verfügt, in das ein hohlzylindrischer Abschnitt des Verschlusskörpers passend eingesetzt ist. Dabei dichtet die Außenfläche des hohlzylindrischen Abschnitts des Verschlusskörpers gegenüber der Innenfläche des hohlzylindrischen Ansatzstücks des Basiskörpers ab, so dass eine erste Dichtfläche geschaffen wird. Zum Öffnen und Schließen des bekannten Drei-Wege-Ventils wird der Verschlusskörper verdreht, so dass der Verschlusskörper eine Hubbewegung ausübt. Dabei greift ein zylindrischer Absperrkörper, der an dem Basiskörper angeordnet ist, in den hohlzylindrischen Abschnitt des Verschlusskörpers ein, so dass eine zweite Dichtfläche geschaffen wird. Das bekannte Drei-Wege-Ventil hat sich in der Praxis bewährt. Als nachteilig erweist sich jedoch, dass zwei Dichtflächen vorhanden sind, die jeweils sicher abdichten müssen. Weiterhin ist nachteilig, dass die zum Verdrehen des Verschlusskörpers aufzubringenden Kräfte verhältnismäßig groß sind, da der hohlzylindrische Abschnitt des Verschlusskörpers relativ fest in dem hohlzylindrischen Ansatzstück des Basiskörpers sitzen muss, um den Verschlusskörper sicher gegenüber dem Basiskörper abzudichten. Erschwerend kommt hinzu, dass beide Teile an einer relativ großen Fläche dicht aneinander liegen.

Der Erfindung liegt die Aufgabe zu Grunde, eine sicher abdichtende und einfach zu handhabende Vorrichtung zu schaffen, die es erlaubt, medizinische Flüssigkeiten zuzuführen, insbesondere in eine Schlauchleitung eines Überleitgeräts zur enteralen und parenteralen Ernährung zu applizieren.

Die Lösung dieser Aufgabe erfolgt erfindungsgemäß mit den Merkmalen des Patentanspruchs 1. Vorteilhafte Ausführungsformen der Erfindung sind Gegenstand der Unteransprüche.

Die erfindungsgemäße Vorrichtung zum Zuführen von Flüssigkeiten zeichnet sich dadurch aus, dass der Basiskörper ein in den Durchgang mündendes hohlzylindrisches Ansatzstück und der Verschlusskörper einen zylindrischen Absperrkörper aufweist, der derart in dem Verschlusskörper angeordnet ist, dass der Absperrkörper in der ersten Position des Verschlusskörpers abdichtend in dem Ansatzstück des Verschlusskörpers sitzt, so dass eine Flüssigkeitsverbindung zwischen dem Einlass des Verschlusskörpers und dem Durchgang des Basiskörpers unterbrochen ist, und in der zweiten Position des Verschlusskörpers nicht in dem Ansatzstück sitzt, so dass die Flüssigkeitsverbindung hergestellt ist. Bei der erfindungsgemäßen Vorrichtung erfolgt daher eine Abdichtung allein dadurch, dass der Absperrkörper des Verschlusskörpers in dem Ansatzstück des Basiskörpers sitzt. Folglich ist nur eine Dichtfläche vorhanden. Dadurch wird das Risiko einer Undichtigkeit verringert. Da die einzige Dichtfläche zwischen Absperrkörper und Ansatzstück relativ klein ausgebildet werden kann, sind verhältnismäßig geringe Kräfte ausreichend, um den Verschlusskörper zwischen der ersten und zweiten Position zu bewegen. Dadurch wird die Handhabung vereinfacht.

Wenn in diesem Zusammenhang von zylindrischen oder hohlzylindrischen Teilen die Rede ist, werden darunter im Wesentlichen zylindrische oder hohlzylindrische Teile verstanden, da die Teile, die gegeneinander abdichten, nicht exakt zylindrisch ausgebildet sein müssen. So können die zylindrischen Teile zur besseren Abdichtung auch eine leichte Konizität aufweisen. Beispielsweise können das hohlzylindrisch Ansatzstück des Basiskörpers und der zylindrische Absperrkörper leicht konisch ausgebildet sein, um eine besonders sichere Abdichtung zu erreichen.

Bei einer bevorzugten Ausführungsform der erfindungsgemäßen Vorrichtung weist der Verschlusskörper einen inneren hohlzylindrischen Abschnitt auf, der das hohlzylindrische Ansatzstück des Basiskörpers umschließt. Dadurch ist der Verschlusskörper an dem Basiskörper festgelegt, wobei der Verschlusskörper um den Basiskörper drehbar ist. Von Vorteil ist, dass nur der zylindrische Absperrkörper des Verschlusskörpers gegenüber dem hohlzylindrischen Ansatzstück des Basiskörpers, nicht jedoch der innere hohlzylindrische Abschnitt des Verschlusskörpers gegenüber dem hohlzylindrischen Ansatzstück des Basiskörpers abgedichtet zu werden braucht. Daher braucht der Verschlusskörper nicht dicht auf dem Basiskörper zu sitzen, wodurch die Reibkräfte relativ gering sind.

Bei einer weiteren besonders bevorzugten Ausführungsform ist der Absperrkörper in dem inneren hohlzylindrischen Abschnitt des Verschlusskörpers mit Stegen befestigt, die einerseits an der Außenfläche des zylindrischen Absperrkörpers und andererseits an der Innenfläche des inneren hohlzylindrischen Abschnitts des Verschlusskörpers angeschlossen sind. Dadurch verbleibt ein ausreichender Querschnitt in dem Verschlusskörper, durch den die medizinische Flüssigkeit strömen kann. Der Absperrkörper kann an dem Verschlusskörper aber beispielsweise auch mit einem scheibenförmigen Körper befestigt sein, in dem ein oder mehrere Durchbrüche vorgesehen sind.

Eine weitere besonders bevorzugte Ausführungsform sieht vor, dass der Basiskörper einen äußeren hohlzylindrischen Abschnitt aufweist, der das hohlzylindrische Ansatzstück des Basiskörpers umschließt, wobei der innere hohlzylindrische Abschnitt des Verschlusskörpers passend in den Spalt zwischen dem äußeren hohlzylindrischen Abschnitt und dem hohlzylindrischen Ansatzstück des Basiskörpers eingesetzt ist. Dadurch wird der Verschlusskörper sicher an dem Basiskörper festgelegt.

Eine weitere besonders bevorzugte Ausführungsform sieht vor, dass der Verschlusskörper einen den äußeren hohlzylindrischen Abschnitt des Basiskörpers übergreifenden äußeren hohlzylindrischen Abschnitt aufweist. Die Hubbewegung beim Drehen des Verschlusskörpers wird vorzugsweise dadurch erreicht, dass der äußere hohlzylindrische Abschnitt des Verschlusskörpers mindesten eine Nut aufweist, in die mindestens ein Ansatz des äußeren hohlzylindrischen Abschnitts des Basiskörpers greift. Dabei kann die Nut über ihren Verlauf entlang des Umfangs unterschiedliche Steigungen haben, was das Öffnungs- oder Schließverhalten beeinflusst. Die Nut erstreckt sich vorzugsweise über einen Umfangswinkel von 90 °, so dass der Verschlusskörper an dem Basiskörper um 90 ° verdreht werden kann. Dieser Drehwinkel ist im Allgemeinen ausreichend, um eine entsprechende Hubbewegung auszuüben. Der Drehwinkel kann aber auch größer oder kleiner sein. Allein entscheidend ist, dass der Verschlusskörper in der ersten Position dicht in dem Ansatzstück sitzt. Vorzugsweise sind an dem Verschlusskörper zwei umfangsmäßig verteilt angeordnete Nuten vorgesehen, in die jeweils ein Ansatz des Basiskörpers greift.

Die erfindungsgemäße Vorrichtung ist vorzugsweise als T-Stück ausgebildet, wobei der Einlass des Verschlusskörpers auf einer Achse liegt, die senkrecht auf der durch den Ein- und Auslass des Basiskörpers verlaufenden Achse steht.

Der Verschlusskörper weist vorzugsweise zwei radial abstehende Griffelemente auf, so dass der Verschlusskörper leicht zu greifen und zu drehen ist. Weiterhin weist der Verschlusskörper vorzugsweise ein Anschlussstück auf, dass als Luer-Lock-Anschlussstück ausgebildet ist, an dem ein entsprechendes Luer-Lock-Anschlussstück angeschlossen werden kann. Anstelle eines Luer-Lock-Anschlussstücks kann aber auch ein Luer-Anschlussstück vorgesehen sein, wenn eine Sicherung der Anschlussstücke durch Verschrauben nicht erforderlich ist. Einlass und Auslass des Basiskörpers sind vorzugsweise als hohlzylindrische Anschlussstücke zum Einschieben einer Schlauchleitung ausgebildet, die mit dem Basiskörper verklebt oder verschweißt werden kann. Es ist aber auch möglich, andere Anschlussstücke an dem Basiskörper vorzusehen.

Die erfindungsgemäße Vorrichtung findet insbesondere zum Applizieren eines Medikaments in die Schlauchleitung eines Überleitgeräts für die enterale oder parenterale Ernährung Verwendung. Es ist aber auch möglich, die erfindungsgemäße Vorrichtung zum Applizieren eines Medikaments in die Schlauchleitung eines Infusionssystems zu verwenden, das nicht der enteralen oder parenteralen Ernährung dient.

Im Folgenden wird ein Ausführungsbeispiel der Erfindung unter Bezugnahme auf die Zeichnungen im Einzelnen erläutert.

Es zeigen:
- Fig. 1: ein Ausführungsbeispiel der erfindungsgemäßen Vorrichtung zum Zuführen von medizinischen Flüssigkeiten in perspektivischer Darstellung, wobei der Durchgang in dem Basiskörper verschlossen ist,
- Fig. 2: die Vorrichtung von Fig. 1, wobei der Durchgang in dem Basiskörper offen ist,
- Fig. 3: die Vorrichtung von Fig. 1 in der Ansicht aus der Richtung des Pfeils III,
- Fig. 4: die Vorrichtung von Fig. 1 in der Draufsicht,
- Fig. 5: die Vorrichtung von Fig. 1 in der Ansicht aus der Richtung des Pfeils V und
- Fig. 6: einen Schnitt durch die Vorrichtung entlang der Linie VI-VI von Fig. 5 in vergrößerter Darstellung.

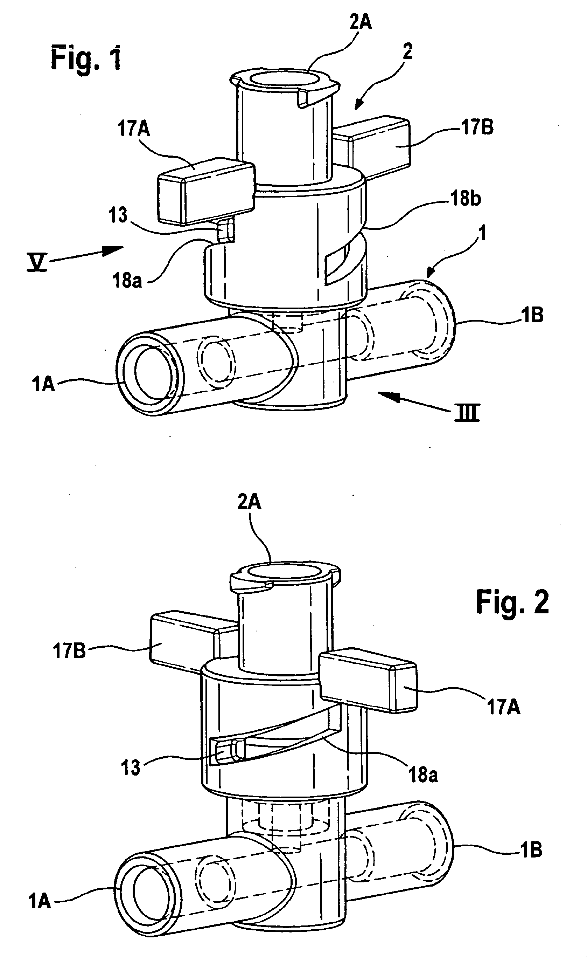

Die Fig. 1 und 2 zeigen in perspektivischer Darstellung ein Ausführungsbeispiel der erfindungsgemäßen Vorrichtung zum Applizieren eines Medikaments in die nicht dargestellte Schlauchleitung eines nicht dargestellten Überleitgeräts zur enteralen oder parenteralen Ernährung. Die Applikationsvorrichtung verfügt über einen Basiskörper 1 und einen Verschlusskörper 2. Basis- und Verschlusskörper 1, 2 sind Spritzgießteile, die in großen Stückzahlen kostengünstig hergestellt werden können.

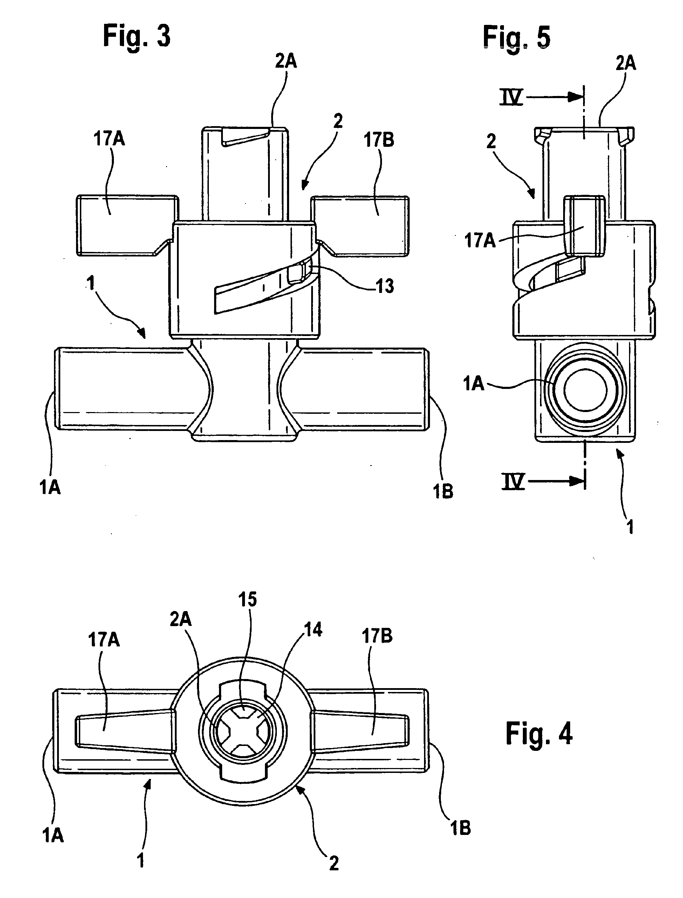

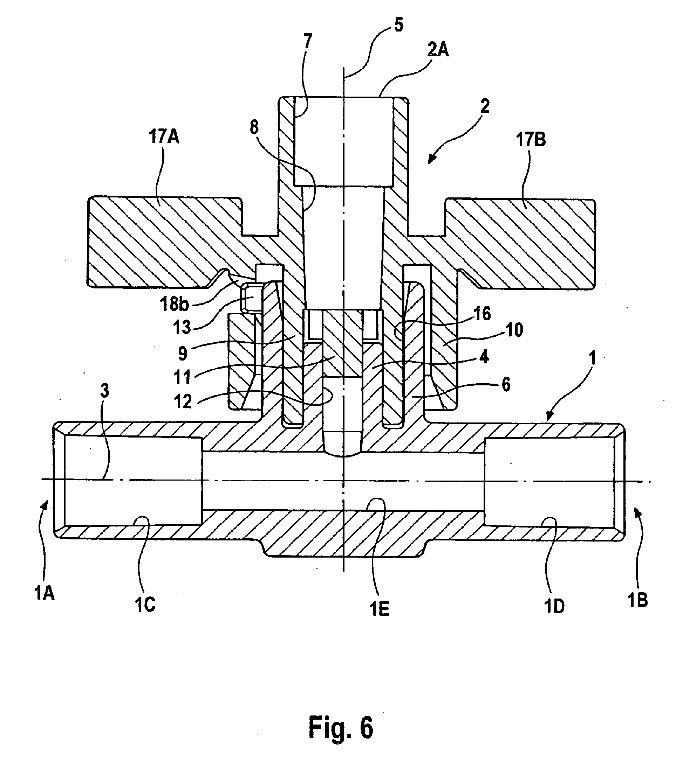

Die Fig. 3 bis 5 zeigen die Applikationsvorrichtung in der Seitenansicht und Draufsicht, während Fig. 6 einen Schnitt durch die Vorrichtung in vergrößerter Darstellung zeigt.

Der Basiskörper 1 weist einen Einlass 1A und einen Auslass 1B auf, die auf einer gemeinsamen Achse 3 liegen. Der Einlass und der Auslass sind als hohlzylindrische Anschlussstücke 1A, 1 B ausgebildet, in die das Ende einer Schlauchleitung eingeschoben werden kann. Dabei kann das jeweilige Schlauchleitungsende in das Anschlussstück 1A, 1 B eingeklebt oder eingeschweißt werden. Vorzugsweise sind die Ausnehmungen 1C, 1 D in den Anschlussstücken konisch. Zwischen dem Einlass 1A und dem Auslass 1B erstreckt sich ein zylindrischer Durchgang 1E geringeren Querschnitts als die konischen Ausnehmungen 1C, 1D der beiden Anschlussstücke 1A, 1B.

In dem Basiskörper 1 ist mittig zwischen den Anschlussstücken 1A, 1B ein hohlzylindrisches Ansatzstück 4 angeformt, das in den Durchgang 1 E des Basiskörpers mündet. Die Längsachse 5 des Ansatzstücks 4 steht senkrecht auf der Achse 3, die durch die Anschlussstücke 1A, 1 B verläuft. Das hohlzylindrische Ansatzstück 4 des Basiskörpers 1 wird unter Bildung eines Spalts 16 von einem äußeren hohlzylindrischen Abschnitt 6 umschlossen, der sich nach oben über das hohlzylindrische Ansatzstück 4 erstreckt.

Der Verschlusskörper 2 weist einen Einlass 2A auf, der als ein hohlzylindrisches Anschlussstück ausgebildet ist, an das eine nicht dargestellte Vorrichtung zum Applizieren eines Medikaments angeschlossen werden kann. Das Anschlussstück 2A kann ein weibliches Luer-Lock-Anschlussstück sein, an das ein männliches Luer-Lock-Anschlussstück angeschlossen werden kann. Derartige Luer-Lock-Anschlussstücke gehören zum Stand der Technik. Das Anschlussstück 2A weist einen zylindrischen Abschnitt 7 auf, um beispielsweise eine orale Spritze oder einen Stufenadapter anschließen zu können, wobei sich an den zylindrischen Abschnitt 7 ein konischer Abschnitt 8 für Luerspritzen anschließt.

Unterhalb des konischen Abschnitts 8 des Anschlussstücks 2A schließt sich ein hohlzylindrischer Abschnitt 9 an, der drehbar in dem Spalt 16 zwischen dem hohlzylindrischen Ansatzstück 4 und dem äußeren hohlzylindrischen Abschnitt 6 des Basiskörpers 1 sitzt.

Der Verschlusskörper 2 weist weiterhin einen äußeren hohlzylindrischen Abschnitt 10 auf, der den äußeren hohlzylindrischen Abschnitt 6 des Basiskörpers 1 übergreift. Der äußere hohlzylindrischen Abschnitt 10 des Verschlusskörpers weist zwei umfangsmäßig verteilt angeordnete Nuten 18a, 18b auf, die sich über einen Umfangswinkel von 90 ° erstrecken. In den beiden Nuten 18a, 18b sitzen jeweils Ansätze, die an den äußeren hohlzylindrischen Abschnitt 6 des Basiskörpers angeformt sind. In den Figuren ist nur einer der beiden Ansätze 13 zu erkennen, da der andere Ansatz auf der anderen Seite liegt.
Die Nuten und Ansätze bilden eine Kulissenführung, die derart ausgebildet ist, dass der Verschlusskörper 2 an dem Basiskörper 1 zwischen den beiden in den Figuren 1 und 2 gezeigten Positionen um 90 ° verdreht werden kann. Dabei übt der Basiskörper eine Hubbewegung aus. In diesen beiden Positionen schlagen die Ansätze des Basiskörpers an dem jeweiligen Ende der Nuten an.

Im Zentrum des unteren hohlzylindrischen Abschnitts 9 des Verschlusskörpers 2 ist ein zylindrischer Absperrkörper 11 angeordnet, mit dem das Lumen 12 des Ansatzstücks 4 verschlossen werden kann. Bei dem zylindrischen Absperrkörper 11 handelt es sich um einen Zapfen, der im Zentrum des Verschlusskörpers 2 angeordnet ist. Der Absperrkörper 11 ist an dem Verschlusskörper 2 mittels vier umfangsmäßig angeordneten Stegen 14 befestigt, die einerseits an der Außenseite des Absperrkörpers 11 und andererseits an der Innenseite des hohlzylindrischen Abschnitts 9 des Verschlusskörpers 2 angeformt sind, so dass die Flüssigkeit durch die zwischen den Stegen 14 liegenden Durchlässe 15 strömen kann (Fig. 4).

Fig. 6 zeigt den Verschlusskörper 2 in der Position, in der das Ansatzstück 4 des Basiskörpers 1 von dem Absperrkörper 11 des Verschlusskörpers 2 verschlossen wird (Fig. 1). Der Absperrkörper 11 sitzt dabei abdichtend in dem Lumen 12 des Ansatzstücks 4. Wenn der Verschlusskörper 2 aus der ersten Position um 90 ° entgegen dem Uhrzeigersinn in die zweite Position verdreht wird (Fig. 2), übt der Verschlusskörper 2 eine Hubbewegung aus. Die Kulissenführung ist derart ausgebildet, dass der Absperrkörper 11 des Verschlusskörpers 2 in der zweiten Position vollständig aus dem Lumen 12 des Ansatzstücks 4 herausgezogen ist. Dadurch wird eine Strömungsverbindung zwischen dem Einlass 2A des Verschlusskörpers 2 und dem Durchgang 1E des Basiskörpers 1 hergestellt. Die Flüssigkeit kann durch den zylindrischen Abschnitt 7, den konischen Abschnitt 8, die Durchlässe 15 und das Lumen 12 in den Durchgang 1E strömen. Wenn der Verschlusskörper 2 wieder im Uhrzeigersinn um 90 ° verdreht wird (Fig. 1), schiebt sich der Absperrkörper 11 wieder in das Lumen 12 des Ansatzstücks 4, so dass die Flüssigkeitsverbindung wieder unterbrochen ist.

Um den Verschlusskörper besser greifen zu können, sind zwei radial abstehende Griffelemente 17A und 17B vorgesehen. Wenn die Strömungsverbindung zwischen dem Einlass 2A des Verschlusskörpers 2 und dem Durchgang 1E des Basiskörpers 1 unterbrochen ist, zeigen die beiden Griffelemente 17A, 17B in Längsrichtung des Basiskörpers (Fig. 1), während die Griffelemente 17A, 17B im rechten Winkel zur Längsrichtung des Basiskörpers angeordnet sind, wenn eine Strömungsverbindung zwischen dem Einlass 2A des Verschlusskörpers 2 und dem Durchgang 1E des Basiskörpers 1 hergestellt ist. Die Strömungsverbindung zwischen dem Einlass 1A und dem Auslass 1 des Basiskörpers 1 bleibt unabhängig von der Position des Verschlusskörpers 2 immer bestehen.

## Patentansprüche

1. Vorrichtung zum Zuführen von medizinischen Flüssigkeiten, insbesondere zum Applizieren eines Medikaments in die Schlauchleitung eines Überleitgeräts für die enterale oder parenterale Ernährung, mit
einem Basiskörper (1), der einen Durchgang (1E) aufweist, der einen Einlass (1A) und einen Auslass (1B) des Basiskörpers miteinander verbindet, und
einem am Basiskörper (1) angeordneten Verschlusskörper (2), der einen Einlass (2A) aufweist,
wobei der Verschlusskörper (2) an dem Basiskörper (1) zwischen einer ersten Position, in der eine Flüssigkeitsverbindung zwischen dem Einlass (2A) des Verschlusskörpers (2) und dem Durchgang (1E) des Basiskörpers (1) unterbrochen ist, und einer zweiten Position drehbar ist, in der die Flüssigkeitsverbindung zwischen dem Einlass (2A) des Verschlusskörpers (2) und dem Durchgang (1E) des Basiskörpers (1) hergestellt ist,
**dadurch gekennzeichnet, dass**
der Basiskörper (1) ein in den Durchgang (1E) mündendes hohlzylindrisches Ansatzstück (4) und der Verschlusskörper (2) einen zylindrischen Absperrkörper (11) aufweist, der derart in dem Verschlusskörper (2) angeordnet ist, dass der Absperrkörper (11) in der ersten Position des Verschlusskörpers (2) abdichtend in dem Ansatzstück (4) des Basiskörpers (1) sitzt, so dass die Flüssigkeitsverbindung zwischen dem Einlass (2A) des Verschlusskörpers (2) und dem Durchgang (1E) des Basiskörpers (1) unterbrochen ist, und der Absperrkörper in der zweiten Position des Verschlusskörpers nicht in dem Ansatzstück sitzt, so dass die Flüssigkeitsverbindung zwischen dem Einlass des Verschlusskörpers und dem Durchgang des Basiskörpers hergestellt ist.

2. Vorrichtung nach Anspruch 1, **dadurch gekennzeichnet, dass** der Verschlusskörper (2) einen inneren hohlzylindrischen Abschnitt (9) aufweist, der das hohlzylindrische Ansatzstück (4) des Basiskörpers (1) umschließt.

3. Vorrichtung nach Anspruch 2, **dadurch gekennzeichnet, dass** der Absperrkörper (11) in dem inneren hohlzylindrischen Abschnitt (9) des Verschlusskörpers (2) mit Stegen (14) befestigt ist, die einerseits an der Außenfläche des Absperrkörpers (11) und andererseits an der Innenfläche des inneren hohlzylindrischen Abschnitts (9) des Verschlusskörpers (2) angeschlossen sind.

4. Vorrichtung nach Anspruch 2 oder 3, **dadurch gekennzeichnet, dass** der Basiskörper (1) einen äußeren hohlzylindrischen Abschnitt (6) aufweist, der das hohlzylindrische Ansatzstück (4) umschließt, wobei der innere hohlzylindrische Abschnitt (9) des Verschlusskörpers (2) passend in den Spalt (16) zwischen dem äußeren hohlzylindrischen Abschnitt (6) des Basiskörpers (1) und dem hohlzylindrischen Ansatzstück (4) des Basiskörpers (1) eingesetzt ist.

5. Vorrichtung nach Anspruch 4, **dadurch gekennzeichnet, dass** der Verschlusskörper (2) einen den äußeren hohlzylindrischen Abschnitt (6) des Basiskörpers (1) übergreifenden äußeren hohlzylindrischen Abschnitt (10) aufweist.

6. Vorrichtung nach Anspruch 5, **dadurch gekennzeichnet, dass** der äußere hohlzylindrische Abschnitt (10) des Verschlusskörpers (2) mindestens eine Nut (18a, 18b) aufweist, in die mindestens ein Ansatz (13) des äußeren hohlzylindrischen Abschnitts (6) des Basiskörpers (1) greift, wobei der Verlauf der Nut (18a, 18b) derart beschaffen ist, dass der Verschlusskörper (2) beim Drehen eine Hubbewegung ausübt.

7. Vorrichtung nach einem der Ansprüche 1 bis 6, **dadurch gekennzeichnet, dass** der Einlass (1A) und Auslass (1B) des Basiskörpers (1) auf einer Achse (3) liegen, wobei der Einlass (2A) des Verschlusskörpers (2) auf einer Achse (5) liegt, die senkrecht auf der durch den Einlass (1A) und Auslass (1 B) des Basiskörpers (1) verlaufenden Achse (3) steht.

8. Vorrichtung nach einem der Ansprüche 1 bis 7, **dadurch gekennzeichnet, dass** der Verschlusskörper (2) zwei Griffelemente (17A, 17B) aufweist, die radial von dem Verschlusskörper abstehen.

9. Vorrichtung nach einem der Ansprüche 1 bis 8, **dadurch gekennzeichnet, dass** der Einlass (2A) des Verschlusskörpers (2) als Luer-Lock-Anschlussstück (2A) ausgebildet ist.

10. Vorrichtung nach einem der Ansprüche 1 bis 9, **dadurch gekennzeichnet, dass** der Einlass (1A) und Auslass (1B) des Basiskörpers (1) als hohlzylindrische Anschlussstücke (1 A, 1B) zum Einschieben einer Schlauchleitung ausgebildet sind.

## Claims

1. Apparatus for supplying medical liquids, in particular for administering a medicine into the hose line of a transfer device for enteral or parenteral nutrition, having
a main body (1) which has a through-passage (1E) which connects an inlet (1A) and an outlet (1B) of the main body together, and
a closure body (2) which is arranged on the main body (1) and has an inlet (2A),
wherein the closure body (2) is rotatable on the main body (1) between a first position, in which a liquid connection between the inlet (2A) of the closure body (2) and the through-passage (1E) in the main body (1) is interrupted, and a second position, in which the liquid connection between the inlet (2A) of the closure body (2) and the through-passage (1E) in the main body (1) is established,
**characterized in that**
the main body (1) has a hollow-cylindrical attachment piece (4) which opens into the through-passage (1E) and the closure body (2) has a cylindrical shut-off body (11) which is arranged in the closure body (2) in such a way that in the first position of the closure body (2) the shut-off body (11) sits in a sealing manner in the attachment piece (4) of the main body (1) and so the liquid connection between the inlet (2A) of the closure body (2) and the through-passage (1E) in the main body (1) is interrupted, and in the second position of the closure body the shut-off body does not sit in the attachment piece and so the liquid connection between the inlet of the closure body and the through-passage in the main body is established.

2. Apparatus according to Claim 1, **characterized in that** the closure body (2) has a hollow-cylindrical portion (9) which encloses the hollow-cylindrical attachment piece (4) of the main body (1).

3. Apparatus according to Claim 2, **characterized in that** the shut-off body (11) is fastened in the inner hollow-cylindrical portion (9) of the closure body (2) by way of ribs (14) which are connected on one side to the outer surface of the shut-off body (11) and on the other side to the inner surface of the inner hollow-cylindrical portion (9) of the closure body (2).

4. Apparatus according to Claim 2 or 3, **characterized in that** the main body (1) has an outer hollow-cylindrical portion (6) which encloses the hollow-cylindrical attachment piece (4), wherein the inner hollow-cylindrical portion (9) of the closure body (2) is inserted with a good fit into the gap (16) between the outer hollow-cylindrical portion (6) of the main body (1) and the hollow-cylindrical attachment piece (4) of the main body (1).

5. Apparatus according to Claim 4, **characterized in that** the closure body (2) has an outer hollow-cylindrical portion (10) that engages over the outer hollow-cylindrical portion (6) of the main body (1).

6. Apparatus according to Claim 5, **characterized in that** the outer hollow-cylindrical portion (10) of the closure body (2) has at least one groove (18a, 18b) into which at least one protrusion (13) of the outer hollow-cylindrical portion (6) of the main body (1) engages, wherein the profile of the groove (18a, 18b) is designed such that the closure body (2) exerts a lifting movement during rotation.

7. Apparatus according to one of Claims 1 to 6, **characterized in that** the inlet (1A) and outlet (1B) of the main body (1) lie on an axis (3), wherein the inlet (2A) of the closure body (2) lies on an axis (5) which is perpendicular to the axis (3) that extends through the inlet (1A) and outlet (1B) of the main body (1).

8. Apparatus according to one of Claims 1 to 7, **characterized in that** the closure body (2) has two gripping elements (17A, 17B) which project radially from the closure body.

9. Apparatus according to one of Claims 1 to 8, **characterized in that** the inlet (2A) of the closure body (2) is in the form of a Luer lock connector piece (2A).

10. Apparatus according to one of Claims 1 to 9, **characterized in that** the inlet (1A) and outlet (1B) of the main body (1) are in the form of hollow-cylindrical connector pieces (1A, 1B) for insertion of a hose line.

## Revendications

1. Dispositif d'alimentation de liquides médicaux, en particulier pour appliquer un médicament dans la conduite tubulaire d'un appareil de transfert pour l'alimentation entérale ou parentérale, comprenant
un corps de base (1) qui présente un passage (1E) qui relie une entrée (1A) et une sortie (1B) du corps de base l'une à l'autre, et
un corps de fermeture (2) disposé sur le corps de base (1), qui présente une entrée (2A),
le corps de fermeture (2) peut tourner sur le corps de base (1) entre une première position, dans laquelle une liaison liquide entre l'entrée (2A) du corps de fermeture (2) et le passage (1E) du corps de base (1) est interrompue, et une deuxième position, dans laquelle la liaison liquide entre l'entrée (2A) du corps de fermeture (2) et le passage (1E) du corps de base (1) est établie,
**caractérisé en ce que**
le corps de base (1) présente une pièce rapportée cylindrique creuse (4) débouchant dans le passage (1E) et le corps de fermeture (2) présente un corps d'arrêt cylindrique (11) qui est disposé dans le corps de fermeture (2) de telle sorte que le corps d'arrêt (11) repose dans la première position du corps de fermeture (2) de manière hermétique dans la pièce rapportée (4) du corps de base (1), de sorte que la liaison liquide entre l'entrée (2A) du corps de fermeture (2) et le passage (1E) du corps de base (1) soit interrompue, et que le corps d'arrêt ne repose pas dans la deuxième position du corps de fermeture dans la pièce rapportée, de sorte que la liaison de liquide entre l'entrée du corps de fermeture et le passage du corps de base soit établie.

2. Dispositif selon la revendication 1, **caractérisé en ce que** le corps de fermeture (2) présente une portion cylindrique creuse interne (9) qui entoure la pièce rapportée cylindrique creuse (4) du corps de base (1).

3. Dispositif selon la revendication 2, **caractérisé en ce que** le corps d'arrêt (11) est fixé dans la portion cylindrique creuse interne (9) du corps de fermeture (2) avec des nervures (14) qui sont raccordées d'une part à la surface extérieure du corps d'arrêt (11) et d'autre part à la surface intérieure de la portion cylindrique creuse interne (9) du corps de fermeture (2).

4. Dispositif selon la revendication 2 ou 3, **caractérisé en ce que** le corps de base (1) présente une portion cylindrique creuse externe (6), qui entoure la pièce rapportée cylindrique creuse (4), la portion cylindrique creuse interne (9) du corps de fermeture (2) étant insérée de manière à s'ajuster dans la fente (16) entre la portion cylindrique creuse externe (6) du corps de base (1) et la pièce rapportée cylindrique creuse (4) du corps de base (1).

5. Dispositif selon la revendication 4, **caractérisé en ce que** le corps de fermeture (2) présente une portion cylindrique creuse externe (10) venant en prise par le dessus avec la portion cylindrique creuse externe (6) du corps de base (1).

6. Dispositif selon la revendication 5, **caractérisé en ce que** la portion cylindrique creuse externe (10) du corps de fermeture (2) présente au moins une rainure (18a, 18b) dans laquelle au moins un insert (13) de la portion cylindrique creuse externe (6) du corps de base (1) vient en prise, l'allure de la rainure (18a, 18b) étant telle que le corps de fermeture (2) exerce un mouvement de levage lors de sa rotation.

7. Dispositif selon l'une quelconque des revendications 1 à 6, **caractérisé en ce que** l'entrée (1A) et la sortie (1B) du corps de base (1) se situent sur un axe (3), l'entrée (2A) du corps de fermeture (2) se trouvant sur un axe (5) qui est perpendiculaire à l'axe (3) s'étendant à travers l'entrée (1A) et la sortie (1B) du corps de base (1).

8. Dispositif selon l'une quelconque des revendications 1 à 7, **caractérisé en ce que** le corps de fermeture (2) présente deux éléments de préhension (17A, 17B) qui font saillie radialement depuis le corps de fermeture.

9. Dispositif selon l'une quelconque des revendications 1 à 8, **caractérisé en ce que** l'entrée (2A) du corps de fermeture (2) est réalisée sous forme de connecteur de verrouillage Luer (2A).

10. Dispositif selon l'une quelconque des revendications 1 à 9, **caractérisé en ce que** l'entrée (1A) et la sortie (1B) du corps de base (1) sont réalisées sous forme de raccords cylindriques creux (1A, 1B) pour l'enfoncement d'une conduite tubulaire.
